(19) Europäisches Patentamt
European Patent Office
Office européen des brevets

(11) **EP 4 516 306 A1**

(12) **EUROPEAN PATENT APPLICATION**
published in accordance with Art. 153(4) EPC

(43) Date of publication:
**05.03.2025 Bulletin 2025/10**

(21) Application number: 23796815.1

(22) Date of filing: **27.04.2023**

(51) International Patent Classification (IPC):
*A61K 35/768* (2015.01)    *A61K 39/12* (2006.01)
*A61P 35/00* (2006.01)    *C12N 7/00* (2006.01)
*C07K 14/005* (2006.01)    *A61K 39/00* (2006.01)

(52) Cooperative Patent Classification (CPC):
**A61K 35/768; A61K 39/00; A61K 39/12;**
**A61P 35/00; C07K 14/005; C12N 7/00;** Y02A 50/30

(86) International application number:
**PCT/KR2023/005736**

(87) International publication number:
**WO 2023/211172 (02.11.2023 Gazette 2023/44)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority:  **27.04.2022  KR 20220052266**

(71) Applicant: SK Bioscience Co., Ltd.
**Bundang-gu, Seongnam-si,**
**Gyeonggi-do, 13494 (KR)**

(72) Inventors:
• **HONG, Seung-hye**
**Seongnam-si, Gyeonggi-do 13494 (KR)**
• **SEO, Ki-Weon**
**Seongnam-si, Gyeonggi-do 13494 (KR)**
• **KIM, Eun-som**
**Seongnam-si, Gyeonggi-do 13494 (KR)**

(74) Representative: **Vossius & Partner**
**Patentanwälte Rechtsanwälte mbB**
**Siebertstrasse 3**
**81675 München (DE)**

Remarks:
The complete document including Reference
Table(s) and the Sequence Listing(s) can be
downloaded from the EPO website

(54) **CHIMERIC ZIKA VIRUS ANTICANCER VACCINE USING BREAST CANCER CELL SUBLINE**

(57)    The present invention relates to a pharmaceutical composition for prevention or treatment breast cancer, containing a chimeric Zika virus obtained through breast cancer cell passages as an active ingredient.

[FIG. 18]

**Description**

**[Technical Field]**

The present invention relates to a pharmaceutical composition for prevention or treatment of breast cancer, containing a chimeric Zika virus obtained through breast cancer cell passages as an active ingredient.

**[Background Art]**

Breast cancer is the most common cancer in women and the second leading cause of death. Risk factors for developing breast cancer include race, age, and mutations in the tumor suppressor genes BRCA-1 and BRCA-2 and p53. Alcohol consumption, a high-fat diet, lack of exercise, exogenous postmenopausal hormones, and ionizing radiation also increase the risk of developing breast cancer.

Common treatments for cancers, including breast cancer, include chemotherapy, radiotherapy, and surgery. However, except for some early-stage cancers, it is difficult to completely remove cancer by surgery or there are side effects due to the cytotoxicity of chemotherapy or radiotherapy. Among the various technologies to supplement this, the strategy of attacking and killing only cancer cells is one of the technologies with great prospects. Among others, an 'oncolytic virus' is a virus that infects and destroys cancer cells based on the cancer cell selection ability inherent in the virus or added artificially, and is a cancer treatment technology that has been in the spotlight for a long time.

In other words, oncolytic viruses are a type of conditionally replication-competent virus that selectively proliferates in tumor cells and kills the tumor cells. During the period from the 1950s to the 1970s, the oncolytic effect of viruses was demonstrated through studies using adenovirus or mumps virus, but oncolytic viruses ultimately received little attention for decades due to the temporary efficacy and toxic side effects. However, after the development of some viruses that were safer than previous viruses, such as thymidine kinase (TK)-deficient variants of herpes simplex virus-1 (HSV-1) and dl1520 (ONYX-015), an adenovirus mutation that selectively proliferates in p53-deficient tumor cells in the 1990s, studies on oncolytic viruses have progressed rapidly (Jagus R, et al., Int. J. Biochem. Cell Biol., (1999) 31: 123-138; Bischoff JR, et al., Science, (1996) 274 (5286): 373-376). To date, about 80 or more types of oncolytic viruses have been developed, and these are expected to be promising tools for cancer treatment.

Zika virus is a virus belonging to the Flaviviridae family, which includes dengue virus, yellow fever virus, West Nile virus, Japanese encephalitis virus, and tick-borne encephalitis virus. Among these, yellow fever virus has been developed and used as a vaccine for a long time, and a live attenuated vaccine was first developed and commercialized around 1930.

**[Disclosure]**

**[Technical Problem]**

There is currently no virus developed specifically to target only breast cancer using Zika virus.

**[Technical Solution]**

An object of the present invention is to provide a pharmaceutical composition for prevention or treatment of breast cancer, containing a chimeric Zika virus as an active ingredient.

Another object of the present invention is to provide a method for preparing the composition.

Still another object of the present invention is to provide a chimeric Zika virus mutant strain obtained using a chimeric gene of an attenuated yellow fever virus strain and Zika virus.

**[Advantageous Effects]**

The present invention can produce an anticancer vaccine that is not only highly effective in treating breast cancer but also has excellent safety by subculturing a chimeric Zika virus (cZIKV), which exhibits an anticancer effect, using breast cancer cells.

**[Brief Description of Drawings]**

FIG. 1 illustrates the construction of a chimeric ZIKA virus in which the main antigen gene of the yellow fever genome is substituted with the main antigen gene of Zika virus;

FIG. 2 is a schematic diagram illustrating a passagingprocess;

FIG. 3 is a schematic diagram illustrating the Viral ToxGlo assay;

FIG. 4 illustrates the results of verifying cZIKV safety through an *in vivo* attenuation test;

FIG. 5 illustrates the experimental design using Balb/c nude (T cell-deficient mice);

FIG. 6 illustrates the test results of the cZIKV infectivity with respect to five normal cell lines;

FIG. 7 illustrates the difference in oncolytic virus infectivity depending on the breast cancer subtype;

FIG. 8 illustrates the results of comparing the infectivity of an oncolytic virus (adapted virus, Bx61) that has undergone passaging with the infectivity of a parental virus (original virus, cZIKV);

FIG. 9 illustrates the results of examining the cytotoxicity of an oncolytic virus (adapted virus, Bx61) that has undergone passaging and a parental virus (original virus, cZIKV);

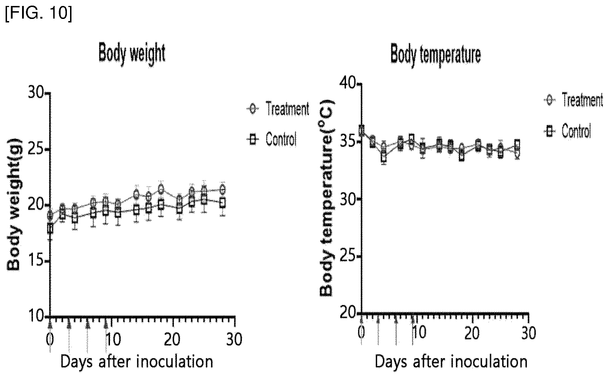

FIG. 10 is a graph illustrating the changes in body weight and body temperature in an oncolytic virus administration group and a control group in a xenograft experiment using MDAMB231, which is highly susceptible to an oncolytic virus;

FIG. 11 is a graph illustrating the change in tumor size in an oncolytic virus administration group and a control group in a xenograft experiment using MDAMB231, which is highly susceptible to an oncolytic virus;

FIG. 12 illustrates the results of tumor histopathological examination of an oncolytic virus administration group and a control group in a xenograft experiment using MDAMB231, which is highly susceptible to an oncolytic virus;

FIG. 13 is a graph illustrating the changes in body weight and body temperature in an oncolytic virus administration group and a control group in a xenograft experiment using BT474, which is hardly susceptible to an oncolytic virus;

FIG. 14 is a graph illustrating the change in tumor size in an oncolytic virus administration group and a control group in a xenograft experiment using BT474, which is hardly susceptible to an oncolytic virus;

FIG. 15 illustrates the results of tumor histopathological examination of an oncolytic virus administration group and a control group in a xenograft experiment using BT474, which is hardly susceptible to an oncolytic virus;

FIG. 16 is a graph illustrating the changes in body weight and body temperature in an oncolytic virus administration group and a control group in a xenograft experiment using the Hs578T cell line, which does not have CPE by an oncolytic virus;

FIG. 17 is a graph illustrating the change in tumor size in an oncolytic virus administration group and a control group in a xenograft experiment using the Hs578T cell line, which does not have CPE by an oncolytic virus;

FIG. 18 illustrates the results of tumor histopathological examination of an oncolytic virus administration group and a control group in a xenograft experiment using the Hs578T cell line, which does not have CPE by an oncolytic virus;

FIG. 19 illustrates the results of histopathology to examine the safety of treatment using an oncolytic virus; and

FIG. 20 illustrates the results of examining the infectivity according to the number of passages using IFA (immunofluorescence assay). For reference, cZIKV: original virus before passage, and MD19 indicates the virus that has been passaged 19 times in MDAMB231, Bx32 indicates the virus passaged 32 times in mixed cells, and Bx61 indicates the virus passaged 61 times in mixed cells, and the passage number increases from left to right.

**[Best Mode for Carrying out the Invention]**

An aspect of the present invention is a pharmaceutical composition for prevention or treatment of breast cancer.

In a specific example, the composition is a pharmaceutical composition for prevention or treatment of breast cancer, containing a chimeric Zika virus formed by introducing a Zika virus antigen gene into a gene of an attenuated yellow fever virus containing SEQ ID NO: 1 as an active ingredient.

The composition according to the preceding specific example, in which the Zika virus antigen gene includes i) a gene encoding a pre-membrane (prM) protein and ii) a gene encoding an envelope (E) protein.

The composition according to any one of the preceding specific examples, in which the Zika virus antigen gene includes i) a gene encoding a pre-membrane (prM) protein of SEQ ID NO: 3 or having 90% or more identity to SEQ ID NO: 3; and ii) a gene encoding an envelope (E) protein of SEQ ID NO: 4 or having 90% or more identity to SEQ ID NO: 4.

The composition according to any one of the preceding specific examples, in which the chimeric Zika virus has ability of specifically inhibiting breast cancer cells through continuous passaging.

The composition according to any one of the preceding specific examples, in which the chimeric Zika virus is obtained through at least 19 passages in MDAMB-231 cells and at least 61 continuous passages in mixed cells consisting of HCC1954, BT474, HCC70, BT20, BT549, and HS578T.

The composition according to any one of the preceding specific examples, in which the chimeric Zika virus includes a polypeptide described by an amino acid sequence of SEQ ID NO: 8 or having 90% or more identity to SEQ ID NO: 8.

The composition according to any one of the preceding specific examples, in which in the chimeric Zika virus, an amino acid corresponding to any one position in the group consisting of 30th, 60th, 122nd, 227th, 427th, 497th, 608th, 657th, 690th, 1464th, 1843rd, 2052nd, 2108th, 2139th, 2150th, 2350th, 2404th, 2416th, 2461st, and 3077th positions based on a sequence represented by SEQ ID NO: 7 is substituted with another amino acid.

The composition according to any one of the preceding specific examples, in which the chimeric Zika virus may include a gene encoding a capsid protein of the yellow fever virus and a gene encoding a non-structural protein, and the capsid (C) protein of the yellow fever virus includes a polypeptide described by an amino acid sequence of SEQ ID NO: 2 or having 90% or more identity to SEQ ID NO: 2.

The composition according to any one of the preceding specific examples, in which in the chimeric Zika virus, amino acids corresponding to 30th and 60th positions based on a sequence represented by SEQ ID NO: 2 are substituted with other amino acids.

The composition according to any one of the preceding specific examples, in which a pre-membrane (prM) protein of the chimeric Zika virus includes a polypeptide described by an amino acid sequence of SEQ ID NO: 3 or having 90% or more identity to SEQ ID NO: 3.

The composition according to any one of the preceding specific examples, in which in the chimeric Zika virus, amino acids corresponding to 122th and 227th positions based on a sequence represented by SEQ ID NO: 3 are substituted with other amino acids.

The composition according to any one of the preceding specific examples, in which an envelope (E) protein of the chimeric Zika virus includes a polypeptide described by an amino acid sequence of SEQ ID NO: 4 or having 90% or more identity to SEQ ID NO: 4.

The composition according to any one of the preceding specific examples, in which in the chimeric Zika virus, an amino acid corresponding to any one position in the group consisting of 427th, 497th, 608th, 657th, and 690th positions based on a sequence represented by SEQ ID NO: 4 is substituted with another amino acid.

The composition according to any one of the preceding specific examples, in which the non-structural protein includes a polypeptide described by an amino acid sequence of SEQ ID NO: 5 or having 90% or more identity to SEQ ID NO: 5.

The composition according to any one of the preceding specific examples, in which in the chimeric Zika virus, amino acids corresponding to 1464th, 1843rd, 2052nd, 2108th, 2139th, 2150th, 2350th, 2404th, 2416th, 2461st, and 3077th positions based on a sequence represented by SEQ ID NO: 5 are substituted with other amino acids.

The composition according to any one of the preceding specific examples, in which the composition is administered intratumorally, intramuscularly, intraperitoneally or intravenously.

Another aspect of the present invention is a method for preparing a pharmaceutical composition for prevention or treatment of breast cancer.

In a specific example, the preparation method includes: (a) continuously subculturing a chimeric Zika virus in MDAMB-231 cells at least 19 times to obtain a virus culture containing a cell-adapted virus; (b) continuously subculturing the culture containing a cell-adapted virus, which is obtained in step (a), in mixed cells consisting of HCC1954, BT474, HCC70, BT20, BT549, and HS578T at least 61 times to obtain a virus culture containing a cell-adapted virus; and (c) selecting and isolating a chimeric Zika virus from the virus culture containing a cell-adapted virus, which is obtained in step (b), by evaluating its ability to specifically inhibit breast cancer cells.

Still another aspect of the present invention is a chimeric nucleic acid molecule.

In a specific example, the chimeric nucleic acid molecule consists of i) a gene encoding a signal peptide located upstream of a gene encoding a pre-membrane (prM) protein including a polypeptide of SEQ ID NO: 6 or having 90% or more identity to SEQ ID NO: 6; ii) a gene encoding a pre-membrane (prM) protein including a polypeptide of SEQ ID NO: 3 or having 90% or more identity to SEQ ID NO: 3; or iii) a gene encoding an envelope (E) protein consisting of a polypeptide of SEQ ID NO: 4 or having 90% or more identity to SEQ ID NO: 4 of Zika virus, which is inserted in place of

a yellow fever virus 17D vaccine strain gene into regions of i) a gene encoding a signal peptide located upstream of a gene encoding a pre-membrane (prM) protein; ii) a gene encoding a pre-membrane (prM) protein; and iii) a gene encoding an envelope (E) protein of a yellow fever virus 17D vaccine strain nucleic acid molecule, in which part or all of a nucleic acid constituting the gene i), ii), or iii) of Zika virus is substituted, deleted, or inserted.

Still another aspect of the present invention is a chimeric Zika virus mutant strain.

In a specific example, the mutant strain is obtained by continuously subculturing a chimeric nucleic acid molecule in breast cancer cells to increase cancer cell adaptability.

The present invention is specifically explained as follows. Meanwhile, each description and each embodiment disclosed in the present invention may also be applied to other descriptions and other embodiments, respectively. In other words, all combinations of the various elements disclosed in the present invention fall within the scope of the present invention. Additionally, the scope of the present invention should not be considered limited by the specific description below. Furthermore, those skilled in the art will recognize, or be able to ascertain using no more than routine experimentation, many equivalents to the specific embodiments of the present invention described herein. Additionally, such equivalents are intended to be included in the present invention.

An aspect of the present invention provides a pharmaceutical composition for prevention or treatment of breast cancer, containing a chimeric Zika virus as an active ingredient. Specifically, a pharmaceutical composition for prevention or treatment of breast cancer, which contains a chimeric Zika virus formed by introducing a Zika virus antigen gene into a gene of an attenuated yellow fever virus containing SEQ ID NO: 1 as an active ingredient, is provided.

The term "chimera" of the present invention may mean a case where cells with different genetic traits exist together in one organism, and particularly in the present invention, means a case where the genetic backbone of one individual is the basis and the genetic information (nucleic acid) of a different individual is inserted to cause new genetic variation. The chimera may be used interchangeably with 'chimeric', 'Zika chimera', 'Zika chimeric', 'chimera Zika virus', 'chimeric Zika virus', or 'cZIKV'.

Zika virus consists of a structure that includes structural proteins and non-structural proteins. Specifically, the structural proteins include the capsid protein (C), pre-membrane protein (precursor membrane protein; prM), and envelope protein (E), and the non-structural proteins include nonstructural proteins NS1 to NS5. For example, the "Zika chimera" or "chimeric Zika virus" of the present invention refers to an acceptor flavivirus in which the genetic backbone has been modified by exchanging at least the sequences of the envelope (E) protein or the pre-membrane (pr-M) and envelope (E) proteins of the acceptor flavivirus with the corresponding sequences of Zika virus. Specifically, the genetic backbone of the acceptor flavivirus may be modified by exchanging the base sequences encoding both the pr-M and E proteins of the acceptor flavivirus with the corresponding sequences of Zika virus. The acceptor flavivirus may be attenuated. The acceptor flavivirus may be the yellow fever (YF) virus, specifically a yellow fever virus 17D vaccine strain.

In the present invention, "chimeric Zika virus" may mean a form in which the yellow fever virus is the backbone and a gene capable of inducing a Zika virus immune response is inserted into the nucleic acid molecule of the yellow fever virus.

Examples of other attenuated YF strains that may be used include, but are not limited to, attenuated YF 17D, YF 17DD, and YF 17D204 (YF-VAX®) viruses. Specifically, the YF 17D strain can be used as a backbone. For example, the YF 17D backbone genome sequence may consist of SEQ ID NO: 1.

As an embodiment according to any one of the preceding embodiments, the Zika virus antigen gene may include i) a gene encoding a pre-membrane (prM) protein and ii) a gene encoding an envelope (E) protein.

The chimeric Zika virus is a virus in which a foreign gene is introduced into the genome of an attenuated yellow fever virus, and may be a virus in which the i) pre-membrane (prM) protein region and ii) envelope (E) protein region of an attenuated yellow fever virus are substituted with those derived from the i) pre-membrane (prM) protein region and ii) envelope (E) protein region of wild-type Zika virus, but is not limited thereto.

In the Example of the present invention, the transmembrane protein region of the 17D YF virus may be maintained as that of the chimeric Zika virus and the pre-membrane (prM) and envelope (E) proteins derived from wild-type Zika virus may be provided as those of the chimeric Zika virus. Specifically, the pre-membrane (prM) protein of the chimeric Zika virus interacts with the envelope (E) protein and may include the whole pre-membrane (prM) protein including the transmembrane protein. In the present invention, the term pre-membrane (prM) may be used interchangeably with a term such as precursor membrane protein.

The pre-membrane (prM) protein of the chimeric Zika virus may include a polypeptide described by an amino acid sequence of SEQ ID NO: 3 or having 90% or more identity thereto.

The envelope (E) protein is a protein located on the outermost surface of flaviviruses, including Zika virus, and is known to be a protein playing a critical role in infection through attachment to host cells. The envelope (E) protein may include the whole envelope protein including the transmembrane protein. The envelope (E) protein may include a polypeptide described by an amino acid sequence of SEQ ID NO: 4 or having 90% or more identity thereto.

In the pre-membrane (prM) protein of the chimeric Zika virus, amino acids corresponding to the 122nd and 227th positions based on the SEQ ID NO: of a sequence represented by SEQ ID NO: 3 may be substituted with other amino acids, and in the envelope (E) protein, an amino acid corresponding to any one position in the group consisting of the 427th, 497th, 608th, 657th, and 690th positions based on the SEQ ID NO: of a sequence represented by SEQ ID NO: 4 may be substituted with another amino acid, but the pre-membrane (prM) protein and envelope (E) protein are not limited thereto.

As an embodiment according to any one of the preceding embodiments, in the present invention, the chimeric Zika virus may include a gene encoding a capsid (C) protein of yellow fever virus and a gene encoding a non-structural protein. Specifically, the capsid (C) protein of the chimeric Zika virus may be the capsid protein of yellow fever virus, and may include a polypeptide described by an amino acid sequence of SEQ ID NO: 2 or having 90% or more identity thereto.

In the capsid (C) protein of the chimeric Zika virus, amino acids corresponding to the 30th and 60th positions based on the SEQ ID NO: of a sequence represented by SEQ ID NO: 2 may be substituted with other amino acids, but the capsid (C) protein is not limited thereto.

Specifically, the non-structural protein of the chimeric Zika virus may be a non-structural protein of yellow fever virus, and may include a polypeptide described by an amino acid sequence of SEQ ID NO: 5 or having 90% or more identity thereto.

In the non-structural protein of the chimeric Zika virus, amino acids corresponding to the 1464th, 1843rd, 2052nd, 2108th, 2139th, 2150th, 2350th, 2404th, 2416th, 2461st, and 3077th positions based on the SEQ ID NO: of a sequence represented by SEQ ID NO: 5 may be substituted with other amino acids, but the non-structural protein is not limited thereto.

For example, an amino acid sequence having 90% or more identity to the amino acid sequence of SEQ ID NO: 2, 3, 4, or 5 of the present invention may be an amino acid sequence having at least 91% or more, 92% or more, 93% or more, 94% or more, 95% or more, 96% or more, 96.26% or more, 97% or more, 97.5% or more, 97.7% or more, 97.8% or more, 98% or more, 98.5% or more, 98.7% or more, 98.8% or more, 99% or more, 99.5% or more, 99.7% or more, 99.8% or more, or more and less than 100% homology or identity to the amino acid sequence of SEQ ID NO: 2, 3, 4, or 5 of the present invention. It is self-evident that a protein having an amino acid sequence in which part of the sequence is deleted, modified, substituted, or added is also included within the scope of the protein to be attenuated in the present invention as long as the amino acid sequence has such homology or identity and exhibits activity the same as or corresponding to that of the capsid (C) protein, pre-membrane (prM) protein, envelope (E) protein and/or non-structural protein.

The capsid (C) protein, pre-membrane (prM) protein, envelope (E) protein or non-structural protein may have a polypeptide described by the amino acid sequence of SEQ ID NO: 2, 3, 4 or 5 or an amino acid sequence having 90% or more identity thereto, or may consist of or consist essentially of the polypeptide. It may also include cases of nonsensical sequence additions (that is, addition of sequences that do not alter the function of the protein to the N-terminus and/or C-terminus of the amino acid sequence) or naturally occurring mutations, silent mutations, or conservative substitutions preceding or following the amino acid sequence of SEQ ID NO: 2, 3, 4 or 5 or having 90% or more identity thereto.

As an embodiment according to any one of the preceding embodiments, for the purposes of the present invention, the chimeric Zika virus including all of the i) gene encoding the pre-membrane (prM) protein and ii) gene encoding the envelope (E) protein derived from Zika virus and the capsid (C) protein gene and non-structural protein gene of yellow fever virus may include a polypeptide described by an amino acid sequence of SEQ ID NO: 7 or having 90% or more identity thereto. An amino acid corresponding to any one position in the group consisting of the 30th, 60th, 122nd, 227th, 427th, 497th, 608th, 657th, 690th, 1464th, 1843rd, 2052nd, 2108th, 2139th, 2150th, 2350th, 2404th, 2416th, 2461st, and 3077th positions based on the SEQ ID NO: of a sequence represented by SEQ ID NO: 7 may be substituted with another amino acid, but the chimeric Zika virus is not limited thereto.

As an embodiment according to any one of the preceding embodiments, the chimeric Zika virus that has undergone passaging may include a polypeptide described by an amino acid sequence of SEQ ID NO: 8 or having 90% or more identity thereto. The chimeric Zika virus may include a polynucleotide encoding an amino acid sequence of SEQ ID NO: 8 or having 90% or more identity thereto. The chimeric Zika virus may, for example, consist of SEQ ID NO: 9.

The "conservative substitution" means substituting one amino acid with another amino acid having similar structural and/or chemical properties. Such amino acid substitution may generally occur based on similarities in the polarity, charge, solubility, hydrophobicity, hydrophilicity, and/or amphipathic nature of the residues. Typically, conservative substitution may hardly affect or may not affect the activity of a protein or polypeptide.

In this application, the term 'homology' or 'identity' means the degree of similarity between two given amino acid sequences or base sequences, and may be represented as a percentage. The terms homology and identity are often used interchangeably.

Sequence homology or identity of conserved polynucleotides or polypeptides is determined by standard alignment algorithms, and a default gap penalty established by the program being used may be used together with this. In practice, homologous or identical sequences can generally hybridize in the whole or part of the sequences under moderate or high stringent conditions. It is self-evident that the hybridization also includes hybridization with polynucleotides containing common codons or codons that take codon degeneracy into account in the polynucleotides.

Whether any two polynucleotide or polypeptide sequences have homology, similarity, or identity may be determined, for example, using a known computer algorithm such as the "FASTA" program, using default parameters such as those in Pearson et al., (1988) [Proc. Natl. Acad. Sci. USA 85]: 2444. The homology, similarity, or identity may be determined using the Needleman-Wunsch algorithm (Needleman and Wunsch, 1970, J. Mol. Biol. 48: 443-453) as performed by the Needleman program of the EMBOSS package (EMBOSS: The European Molecular Biology Open Software Suite, Rice et al., 2000, Trends Genet. 16: 276-277) (version 5.0.0 or later) (including the GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego, 1994, and [CARILLO ETA/.] (1988) SIAM J Applied Math 48: 1073). For example, BLAST from the National Center for Biotechnology Information Database, or ClustalW can be used to determine homology, similarity, or identity.

The homology, similarity, or identity of polynucleotides or polypeptides may be determined by comparing sequence information, for example, using the GAP computer program such as Needleman et al. (1970), J Mol Biol. 48:443, for example, as reported in Smith and Waterman, Adv. Appl. Math (1981) 2:482. In summary, in the GAP program, the homology, similarity or identity may be defined as the value obtained by dividing the number of similarly arranged symbols (namely, nucleotides or amino acids) by the total number of symbols in the shorter of the two sequences. The default parameters for the GAP program may include (1) a binary comparison matrix (containing values of 1 for identity and 0 for non-identity) and a weighted comparison matrix of Gribskov et al (1986) Nucl. Acids Res. 14: 6745 (or EDNAFULL (EMBOSS version of NCBI NUC4.4) permutation matrix) as disclosed by Schwartz and Dayhoff, eds., Atlas Of Protein Sequence And Structure, National Biomedical Research Foundation, pp. 353-358 (1979); (2) a penalty of 3.0 for each gap and an additional penalty of 0.10 for each symbol in each gap (or a gap opening penalty of 10 and a gap extension penalty of 0.5); and (3) no penalty for a terminal gap.

In the present invention, the term "corresponding to" refers to an amino acid residue or a nucleotide residue at a position listed in a polypeptide or polynucleotide, or an amino acid residue or nucleotide residue that is similar, identical, or homologous to a residue listed in a polypeptide or polynucleotide. Identifying the amino acid or nucleotide at a corresponding position may involve determining a particular amino acid or nucleotide of a sequence that references a particular sequence. As used herein, "corresponding region" generally refers to a similar or corresponding position in a related protein or reference protein.

For example, any amino acid sequence can be aligned with SEQ ID NO: 1, 2, 3, 4, or 5, and based on this, each amino acid residue of the amino acid sequence can be numbered by referring to the numerical position of the amino acid residue corresponding to the amino acid residue of the SEQ ID NO: 1, 2, 3, 4 or 5. For example, a sequence alignment algorithm such as that described in the present invention can identify the positions of amino acids or positions where modifications, such as substitutions, insertions, or deletions occur by comparing the sequence with a query sequence (also referred to as a "reference sequence").

For such alignment, for example, the Needleman-Wunsch algorithm (Needleman and Wunsch, 1970, J. Mol. Biol. 48: 443-453), the Needleman program of the EMBOSS package (EMBOSS: The European Molecular Biology Open Software Suite, Rice et al., 2000, Trends Genet. 16: 276-277) and the like may be used, but the alignment is not limited thereto, and any sequence alignment program known in the art, a pairwise sequence comparison algorithm, and the like may be appropriately used.

As an embodiment according to any one of the preceding embodiments, the chimeric Zika virus of the present invention may have the ability of specifically inhibiting breast cancer cells through continuous passaging. For example, the chimeric Zika virus may be obtained through at least 19 passages in MDAMB-231 cells and at least 61 continuous passaging in mixed cells consisting of HCC1954, BT474, HCC70, BT20, BT549, and HS578T, but is not limited thereto. Depending on the type of breast cancer cell line targeted, the passaging order and/or number of times passaged may be adjusted.

The chimeric Zika virus of the present invention is an oncolytic virus, which exhibits properties of low infection and proliferation in normal cells and relatively high infection and proliferation in cancer cells. Using this principle, the chimeric Zika virus can be used in treatment to minimize damage to normal cells and selectively kill only cancer cells.

In the Example of the present invention, the presence or absence of infectivity was examined using primary cells of the brain, testis, skin, breast, and lung, and as a result, infectivity was not observed in normal cells.

In the Example of the present invention, the chimeric Zika virus was passaged in breast cancer cells, and as a result, it was found that the initial chimeric Zika virus (cZIKV) had a weak level of infectivity with respect to cancer cells, but as the number of passages increased, the infectivity with respect to breast cancer cells with initially low susceptibility (for example, BT20 and BT474) increased.

Specifically, it has been found that the adaptation to breast cancer cells increases by subculturing the chimeric Zika virus in breast cancer cells, and the chimeric Zika virus does not affect normal tissue cells without mutations but selectively infects four different subtypes of breast cancer cells, which are classified based on the presence or absence of estrogen receptors, progesterone receptors, and HER2 genes, and can suppress tumors, thereby completing the present invention.

The pharmaceutical composition of the present invention may additionally contain a "pharmaceutically acceptable salt." The 'pharmaceutically acceptable salt' refers to a salt in the form of being used pharmaceutically among salts in which cations and anions are bonded by electrostatic attraction, and typically may be a metal salt, a salt with an organic base, a salt with an inorganic acid, a salt with an organic acid, a salt with a basic or acidic amino acid, and the like. For example, the metal salt may be alkali metal salts (sodium salts, potassium salts, and the like), alkaline earth metal salts (calcium salts, magnesium salts, barium salts, and the like), aluminum salts, and the like; the salt with an organic base may be salts of triethylamine, pyridine, picoline, 2,6-lutidine, ethanolamine, diethanolamine, triethanolamine, cyclohexylamine, dicyclohexylamine, N,N-dibenzylethylenediamine and the like; the salts with an inorganic acid may be salts of hydrochloric acid, hydrobromic acid, nitric acid, sulfuric acid, phosphoric acid and the like; the salts with an organic acid may be salts of formic acid, acetic acid, trifluoroacetic acid, phthalic acid, fumaric acid, oxalic acid, tartaric acid, maleic acid, citric acid, succinic acid, methanesulfonic acid, benzenesulfonic acid, p-toluenesulfonic acid and the like; the salt with a basic amino acid may be salts of arginine, lysine, ornithine and the like; and the salt with an acidic amino acid may be salts of aspartic acid, glutamic acid and the like.

The term "treatment" of the present invention refers to clinical intervention to alter the natural process of an individual or cell to be treated, and this can be done while a clinical pathological condition is progressing or to prevent this. The desired therapeutic effects include preventing the occurrence or recurrence of the disease, alleviating symptoms,

EP 4 516 306 A1

reducing all direct or indirect pathological consequences of the disease, preventing metastasis, slowing the progression of the disease, mitigating or temporarily alleviating the disease status, improving the course of the disease, or improving the prognosis. In the present invention, the treatment includes all actions that improve the course of breast cancer by administering the composition. The term "prevention" of the present invention means all actions that inhibit or delay the onset of breast cancer by administering the composition according to the present invention.

In the Example of the present invention, the anticancer effect was examined using three types of cells with different virus susceptibilities, and as a result, it was found that there was no change in body temperature and body weight, and the tumor size decreased and gradually disappeared in the oncolytic virus administration group. The histopathological examination of tumors also demonstrated that central necrosis was observed in the tumors of the treatment group. Through this, it can be seen that breast cancer can be effectively treated by administering a pharmaceutical composition for prevention or treatment of breast cancer containing the chimeric Zika virus of the present invention as an active ingredient.

The pharmaceutical composition of the present invention may additionally contain an appropriate carrier, excipient, or diluent commonly used in the preparation of pharmaceutical compositions. The composition containing a pharmaceutically acceptable carrier may be various oral or parenteral formulations. In a case where the composition is formed into a preparation, the preparation may be prepared using diluents or excipients such as fillers, extenders, binders, wetting agents, disintegrants, and surfactants that are commonly used. Solid preparations for oral administration may include tablets, pills, powders, granules, capsules, and the like. These solid preparations may be prepared by mixing one or more compounds with at least one or more excipients, for example, starch, calcium carbonate, sucrose or lactose, and gelatin. In addition to simple excipients, lubricants such as magnesium stearate and talc may also be used. Liquid preparations for oral administration include suspensions, oral solutions, emulsions, and syrups, and may contain various excipients, for example, wetting agents, sweeteners, flavoring agents, and preservatives in addition to the commonly used simple diluents, such as water and liquid paraffin. Preparations for parenteral administration may include sterile aqueous solutions, non-aqueous solutions, suspensions, emulsions, lyophilized preparations, and suppositories. As non-aqueous solvents and suspending agents, propylene glycol, polyethylene glycol, vegetable oils such as olive oil, injectable esters such as ethyl oleate, and the like may be used. As the bases for suppositories, Witepsol, macrogol, Tween 61, cocoa butter, laurin butter, glycerogelatin and the like may be used.

The pharmaceutical composition of the present invention may have any one formulation selected from the group consisting of, but not limited to, tablets, pills, powders, granules, capsules, suspensions, oral solutions, emulsions, syrups, sterile aqueous solutions, non-aqueous solutions, suspensions, emulsions, lyophilized preparations, and suppositories.

The pharmaceutical composition of the present invention can be administered to a subject by any appropriate method. As the route of administration, the pharmaceutical composition may be administered via various routes, either oral or parenteral, as long as it can reach the target tissue.

In the present invention, the term "subject" means all animals, such as rats and livestock, including humans that have developed or are capable of developing breast cancer. As a specific example, the subject may be a mammal, including a human.

The pharmaceutical composition can be appropriately administered to a subject depending on the purpose or need by a conventional method, route of administration, and dose used in the art. Examples of the route of administration include parenteral, subcutaneous, intraperitoneal, intrapulmonary, and intranasal administration, and parenteral injection includes intramuscular, intravenous, intraarterial, intraperitoneal, or subcutaneous administration. The appropriate dose and number of times administered may be selected according to methods known in the art. The amount of the pharmaceutical composition of the present invention actually administered and the number of times administered may be appropriately determined by various factors such as the type of symptoms to be treated, route of administration, sex, health status, diet, age and body weight of the subject, and severity of the disease.

As an embodiment according to any one of the preceding embodiments, the composition of the present invention is an attenuated virus vaccine having a chimeric Zika virus structure and can thus have high stability.

The term "attenuation" of the present invention means, but is not limited to, the absence of replication and proliferation ability in normal cells, and includes all meanings known in the art.

In the Example of the present invention, YF17D (yellow fever 17D) and ZIKA exhibited replication ability in mice, but an oncolytic virus, which was a chimeric form of the two viruses, hardly exhibited replication and proliferation ability, verifying its safety.

Through this, it can be seen that not only breast cancer can be effectively treated by administering a pharmaceutical composition containing the chimeric Zika virus of the present invention as an active ingredient, but also the pharmaceutical composition is greatly safe.

Still another aspect of the present invention provides a method for preparing a composition for prevention or treatment breast cancer. Specifically, the method may include (a) continuously subculturing a chimeric Zika virus in MDAMB-231 cells at least 19 times to obtain a virus culture containing a cell-adapted virus; (b) continuously subculturing the culture containing a cell-adapted virus, which is obtained in step (a), in mixed cells consisting of HCC1954, BT474,

8

HCC70, BT20, BT549, and HS578T at least 61 times to obtain a virus culture containing a cell-adapted virus; and (c) selecting and isolating a chimeric Zika virus from the virus culture containing a cell-adapted virus, which is obtained in step (b), by evaluating the ability of specifically inhibiting breast cancer cells.

Still another aspect of the present invention provides a chimeric nucleic acid molecule consisting of i) a gene encoding a signal peptide located upstream of a gene encoding a pre-membrane (prM) protein including a polypeptide of SEQ ID NO: 6 or having 90% or more identity to SEQ ID NO: 6; ii) a gene encoding a pre-membrane (prM) protein including a polypeptide of SEQ ID NO: 3 or having 90% or more identity to SEQ ID NO: 3; or iii) a gene encoding an envelope (E) protein consisting of a polypeptide of SEQ ID NO: 4 or having 90% or more identity to SEQ ID NO: 4 of Zika virus, which is inserted in place of

a yellow fever virus 17D vaccine strain gene into regions of i) a gene encoding a signal peptide located upstream of a gene encoding a pre-membrane (prM) protein; ii) a gene encoding a pre-membrane (prM) protein; and iii) a gene encoding an envelope (E) protein of a yellow fever virus 17D vaccine strain nucleic acid molecule, in which part or all of a nucleic acid constituting the gene i), ii), or iii) of Zika virus is substituted, deleted, or inserted.

Still another aspect of the present invention provides a chimeric Zika virus mutant strain obtained by continuously subculturing the chimeric nucleic acid molecule in breast cancer cells to have increased cancer cell adaptability.

The term "variant" in the present invention refers to a polypeptide in which one or more amino acids are conservatively substituted and/or modified so that the amino acid sequence is different from that of the variant before variation but the functions or properties are maintained. Such a variant can generally be identified by modifying one or more amino acids in the amino acid sequence of the polypeptide and evaluating the properties of the modified polypeptide. In other words, the ability of the variant may be increased, unchanged, or decreased compared to that of the polypeptide before variation. Some variants may include variants in which one or more portions, such as the N-terminal leader sequence or the transmembrane domain, are deleted. Other variants may include variants in which some portions are deleted from the N- and/or C-terminus of the mature protein. The term "variant" may be used interchangeably with terms such as modification, modified polypeptide, modified protein, mutant, mutein, and divergent, and is not limited thereto as long as it is a term used to mean variation. For the purpose of the present invention, in the variant, an amino acid corresponding to SEQ ID NO: 7 or any one position in the group consisting of the 30th, 60th, 122nd, 227th, 427th, 497th, 608th, 657th, 690th, 1464th, 1843rd, 2052nd, 2108th, 2139th, 2150th, 2350th, 2404th, 2416th, 2461st, and 3077th positions of an amino acid sequence of SEQ ID NO: 7 may be substituted with another amino acid.

The variant may include deletions or additions of amino acids that minimally affect the properties and secondary structure of the polypeptide. For example, a signal (or leader) sequence that is involved in protein translocation co-translationally or post-translationally may be conjugated to the N-terminus of the variant. The variant may be conjugated to other sequences or linkers so as to be identified, purified, or synthesized.

## [Detailed Description of the Invention]

Hereinafter, the present invention will be described in more detail with reference to the Examples. These Examples are intended only to illustrate the present invention and the scope of the present invention is not to be construed as being limited by these Examples.

### Experimental Example 1. Construction of chimeric Zika virus (cZIKV)

### 1-1. Preparation of Zika virus

ATCC VR-1843 wild-type Zika virus was prepared. The sequence can be easily acquired through GenBank No: KU365778.1 (Virus strain; BeH819015).

### 1-2. Preparation of yellow fever vaccine strain, 17D virus

Yellow fever vaccine strain, 17D virus was prepared. The 17D virus can be easily acquired through GenBank No: X03700.1.

### 1-3. Design and synthesis of chimeric Zika virus gene

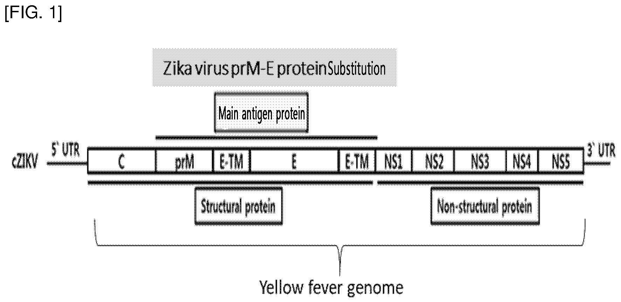

A chimeric Zika virus having the yellow fever genome as the backbone was selected as a candidate for an anticancer vaccine. A chimeric ZIKA virus was constructed by substituting the main antigen gene of the yellow fever genome with the main antigen gene of Zika virus (FIG. 1).

The specific experimental method is the same as that disclosed in Korean Patent Application No. 10-2019-0127605 filed with the Korean Intellectual Property Office on May 3, 2019 by the inventors of the present invention.

**Experimental Example 2. Test of chimeric Zika virus (cZIKV) infectivity with respect to normal cells (5 types) and various cancer cells (5 types)**

**2-1. Preparation of normal cells and cancer cell lines**

In order to examine the Zika virus infectivity in normal cell lines, five types of normal cell lines (brain, testis, skin, breast, and lung) were purchased and cultured using media appropriate for the cell lines. In addition, cell lines representing five types of cancer with high incidence were purchased and cultured using media appropriate for the cell lines.

**2-2. Preparation of eight types of cell lines by breast cancer subtype and examination of cZIKV infectivity with respect to cancer cells**

In order to examine the Zika virus infectivity by breast cancer subtype, eight types of human-derived breast cancer cell lines were purchased (Korean Cell Line Bank and ATCC) and cultured using appropriate media.
The cells cultured above were seeded at 5E5 ($5 \times 10^5$) cells/well in a 6-well plate. The next day, the prepared cells were carefully washed once with FBS-free medium to prevent the cells from falling off. The medium was removed, and all the cells were treated with cZIKV at 1 MOI, and then adsorbed in a 37°C incubator for 1 hr. The infected virus culture was removed, appropriate medium for each cell was added by 3 mL, and the cells were cultured in a 37°C incubator for 3 days.
The culture was removed, and 100% MeOH solution at -20°C was used at 2 mL/well to fix the cells, and the cells were left to stand at RT for 1 hr. Afterwards, the fixing solution was removed, and washing with DPBS was performed three times. The 1st antibody (anti-Flavivirus Group Antigen Antibody, clone D1-4G2-4-15, mouse IgG2) was diluted with DPBS at 1 : 3000 and used at 0.5 mL/well, and the reaction was conducted on a rocker at RT for 1 hr. Washing with DPBS was performed three times. The 2nd antibody (anti-mouse Alexa488) was diluted at 1 : 3000 and used at 0.5 mL/well, and the reaction was conducted on a rocker at RT for 1 hr. At this time, foil was covered over the plate to block light. Washing with DPBS was performed three times. The degree of fluorescence brightness was observed using a fluorescence microscope in a darkroom, or imaging analysis was performed using a Cytation Reader.

**Experimental Example** 3. **Adaptive passaging of** oncolytic **Zika virus 3-1. MDAMB231 adaptation**

MDAMB231 was seeded in a 6-well plate at 3E5 cells/well. The next day, the cells that had reached 80% confluency were infected with cZIKV at 1 MOI. After 1 hour, the infected solution was removed and 3 mL of DMEM (10% FBS, 1% P/S) medium was added. Cytopathic effects can be observed after 2 days. On the third day, the medium was found to be yellowish, and the cells were scraped. The cell lysate and culture were transferred to a 15 mL tube and centrifuged at 3000 rpm for 5 min. The supernatant was taken and divided into 0.5 mL portions in cryovials, which were defined as passage 1. The passaging was repeated 19 times in the same manner as the above process (however, the amount of virus used for reinfection was set to 1 mL since titration was not possible each time). Afterwards, the divided virus was stored at -80°C.

**3-2. Mixed breast cancer cell adaptation**

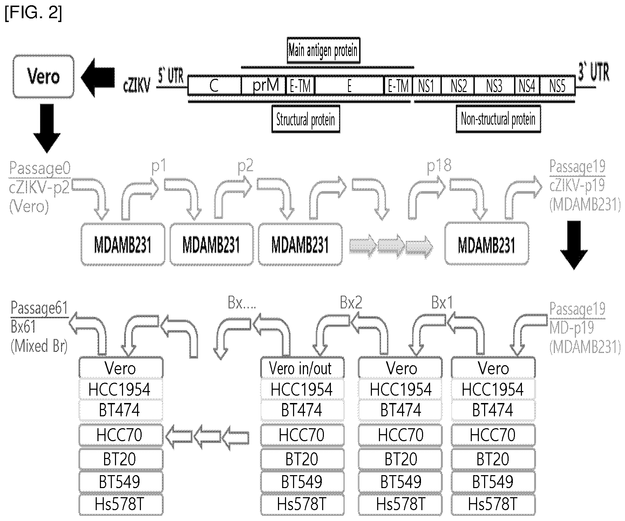

Each of six types of breast cancer cell lines (HCC1954, BT474, HCC70, BT20, BT549, and Hs578T) was seeded at 1E5 cells/well in one well of a 6-well plate (total 6E5 cells/well). The next day, the cells that had reached 80% confluency were infected with the MDAMB231-p19 virus at 1 MOI. The subsequent method was the same as that in Experimental Example 2-2, and passaging was repeated 61 times (labeling the virus Bx61) (FIG. 2).

**3-3. Characterization using adapted virus specific to cancer cells**

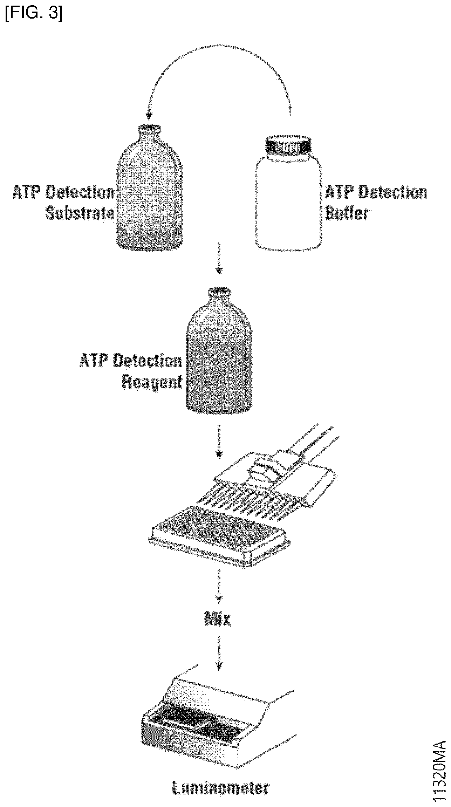

In order to compare the infectivity of cZIKV and Bx61 that had undergone passaging, the infectivity test performed in Experimental Example 2-2 was performed again.
In order to examine the difference in oncolytic effect by cell line, a CPE (cytopathic effect) test was performed, and comparison was performed using cell viability. At this time, the ToxGlo assay kit was used to measure cell viability. Six types of cells (Vero, MCF7, MDAMB231, BT20, Hs578T, and HMEc) were seeded at 2E4 cells/well in a white/clear bottom 96-well plate and treated with two types of viruses (cZIKV and Bx61) at MOIs of 0.1, 1, and 5 (treated with medium as a negative control). At 5 DPI after treatment with the virus, the cell medium was removed. With the lamp of the BSC turned off and in the dark (to prevent decomposition of the substrate by light), the ATP detection substrate and detection buffer were mixed as illustrated in FIG. 3. The mixed solution was put in the wells by 100 μL, and the wells were left to stand in a light blocked state for at least 10 min. At this time, it was carefully checked that there were no bubbles, and then the degree of luminescence was compared using a luminometer.

### 3-4. q-PCR method for quantifying oncolytic Zika virus

Viral RNA was isolated and purified from 200 μL of Zika virus solution using a viral RNA purification kit (PureLink viral RNA/DNA Mini Kit). For analysis, a q-PCR analysis kit (zika virus polyprotein gene genesig advanced kit) and a reverse transcriptase premix kit (Genesig OasigTM Lyophilsed onestep or precisionTM onestep 2X qRT-PCR master mix) were mixed with the sample for preparation.

The prepared standard sample and virus sample were reacted using QuantStudio3 qPCR equipment under the conditions shown in Table 1 below.

[Table 1]

| qPCR reaction conditions | | | |
|---|---|---|---|
| **Enzyme activation** | **Denaturation** | **Denaturation** | **Data collection** |
| 1 cycle | | 40 cycles | |
| 48°C | 95°C | 95°C | 60°C |
| 15 min | 10 sec | 15 sec | 1 min |

### 3-5. Oncolytic Zika virus full-length sequence analysis method

Viral RNA was extracted from 200 μL of virus solution. Viral RNA was prepared using the PureLink™ Viral RNA/DNA mini kit (final elution was set to 25 μL). The concentration of the virus solution used for RNA isolation was 1E7 PFU/mL in the case of oncolytic Zika virus. cDNA synthesis from the extracted viral RNA and gene-specific PCR were performed in one step using a kit (SuperScript One-Step RT-PCR). At this time, RT-PCR and PCR were performed for four overlapping fragment genes (approximately 2.5 to 3 kb in length) encompassing the full-length Zika virus gene. The primers are shown in Table 2 below, and cZik1-F and cZik1-R (2498) were used for fragment 1, cZik2-F and cZik2-R (5385) for fragment 2, cZik3-F and cZik3-R (8205) for fragment 3, and cZika4-F and cZik4-R for fragment 4. PCR was performed under the conditions in Table 3 below and electrophoresis was performed.

[Table 2]

| RT-PCR primer sequences | | |
|---|---|---|
| No. | Primer name | Primer sequence |
| 10 | 5' UTR_For | 5'-TAA TAC GAC TCA CTA TAG GAG TAA ATC CTG TGT GCT-3' |
| 11 | 5' UTR_Rev | 5'-CAG CAT AGG GCA TTC ATA GCT CAT GGT GGC-3' |
| 12 | 3' UTR_For | 5'-GTC ATC CAT CGT ATC CGA ACG CTG ATT GGA-3' |
| 13 | 3' UTR_Rev | 5'-AGT GGT TTT GTG TTT GTC ATC CAA AGG TCT-3' |
| 14 | NS5 region_For | 5'-AAA TGA AAG TGC AAC AAT CTT GAT GAC-3' |
| 15 | NS5 region_Rev | 5'-TGT TCA GGA CCA CCA CAC TCT TTC CA-3' |
| 16 | cZik1-F(1) | 5'-AGT AAA TCC TGT GTG CTA ATT GAG GTG CAT TG-3' |
| 17 | cZik1-R(2498) | 5'- AGC AGA GAC GGC TGT GGA TAA GAA G-3' |
| 18 | cZik2-F(2015) | 5'-CAG ATG GCG GTG GAC ATG CAA ACT CTG ACC CC-3' |
| 19 | cZik2-R(5385) | 5'-CTG CCG TGA GCG GAA AAA GCC TGT GTG TGG AA-3' |
| 20 | cZik3-F(4988) | 5'-GAAATC GGG GCT GTC GCT CTT GAC TAT CCG AG-3' |
| 21 | cZik3-R(8205) | 5'-CCA CAA GCC AGC CAT TTT TCT ACA GTA TCA AG-3' |
| 22 | czika4-F(7894) | 5'-CAA GCT GGA AGG TAG GGT GAT TGA CCT GGG GT-3' |
| 23 | cZik4-R(10907) | 5'-AGT GGT TTT GTG TTT GTC ATC CAA AGG TCT GC-3' |

(continued)

| RT-PCR primer sequences | | |
|---|---|---|
| No. | Primer name | Primer sequence |
| 24 | cz-seq3 | 5'-CAA AAA AGG TGAAGC ACG GAG ATC T-3' |
| 25 | cz-seq4 | 5'-TGG ATG TGG ACT TTT TGG CAAAGG GA-3' |
| 26 | cz-seq5 | 5'-CAG ATG GCG GTG GAC ATG CAA ACT CTG-3' |
| 27 | cz-seq6 | 5'-AGA CTC TGA TGA CTG GCT GAA CAA GTA-3' |
| 28 | cz-seq7 | 5'-ATG ATC CAC ACC TTG GAG GCA TTA GAT TA-3' |
| 29 | cz-seq8 | 5'-CGT TGA TTG CTG CCT TTT CAA TCA GAC CAG-3' |
| 30 | cz-seq9 | 5'-GGT TAG CGT GGC TGG GAG GGT GGA TGG GC-3' |
| 31 | cz-seq10 | 5'-GAAATC GGG GCT GTC GCT CTT GAC TAT CCG-3' |
| 32 | cz-seq11 | 5'-AAA TGA AAG TGC AAC AAT CTT GAT GAC-3' |
| 33 | cz-seq12 | 5'-GAT GGA GAC TCA TAC TAC TAT TCT GAG C-3' |
| 34 | cz-seq13 | 5'-CTC TAG GGC TTA CCG CAA TGC ACT ATC A-3' |
| 35 | cz-seq14 | 5'-GTG ATG CCT CTG CTC TGT GGC ATA GGG TG-3' |
| 36 | cz-seq15 | 5'-CAA GCT GGA AGG TAG GGT GAT TGA CCT GG-3' |
| 37 | cz-seq16 | 5'-GAG AGG ATA AAA TCT GAG TAC ATG ACC-3' |
| 38 | cz-seq17 | 5'-CCT GGC TGC AAT GGA TGG TGG TGG ATT C-3' |
| 39 | cz-seq18 | 5'-TGT GCC CTT CTG TTC CCA CCA CTT CCA TGA-3' |
| 40 | cz-seq19 | 5'-GTG GAA CAG AGT ATG GAT AAC CAA CAA C-3' |
| 41 | cz-seq20 | 5'-ACT CCC CAC AAC CTG AAA CCG GGA TAT AA-3' |

[Table 3]

| RT-PCR conditions | |
|---|---|
| Component | Volume ($\mu$L) |
| 2X reaction mix | 25 |
| Template RNA | 5 |
| Forward primer (10 pmole/$\mu$L) | 1 |
| Reverse primer (10 pmole/$\mu$L) | 1 |
| SuperScript™ III RT/Platinum™ Taq Mix | 1 |
| RNase-free water | 17 |
| Total | 50 |

## Experimental Example 4. Safety verification of chimeric Zika virus (cZIKV)

The cZIKV according to the present invention is an attenuated virus vaccine having a chimeric virus structure, and it was intended to verify the safety of cZIKV compared to that of the two viruses contained in the cZIKV construct.

Specifically, 7-week-old Balb/c female mice were purchased and used in the experiment after an acclimatization period of approximately one week. Purified samples to be applied to the animal experiment were prepared at a concentration of cZIKV ($4 \times 10^5$ pfu/ml) (5 groups total, 5 animals per group - group 1: VP-SFM 400 $\mu$l, group 2: 1E2, group 3: 1E3, group 4: 1E4, and group 5: 1E5).

Some of the injection samples were mixed with an equal volume of adjuvant (alum hydroxide) (vortexed for 1 hr) for preparation. The injection method was intravenous injection, in which the injection sample was administered into the hind limb muscles of mice using an insulin syringe. Infection was performed once, and the body weight of the mouse was measured daily. For each group, blood was collected by 100 $\mu$l via the orbital vein over 10 days after infection. Whole blood was collected on day 28. The collected blood sample was pooled and 100 μl was used. The collected blood was reacted at room temperature for approximately 1 to 2 hours to coagulate the blood. Once the blood had coagulated, the tube was transferred to ice, and the reaction was conducted for 1 hr. Centrifugation was performed at 13,000 rpm and 4°C for 15 min. As much serum as possible was collected, then centrifugation was performed again at 13,000 rpm for 1 min, and the serum from the upper layer was transferred to a new tube and stored frozen at - 20°C. The stored serum was thawed, and q-PCR was performed. The Q-PCR was performed in the same manner as the above method.

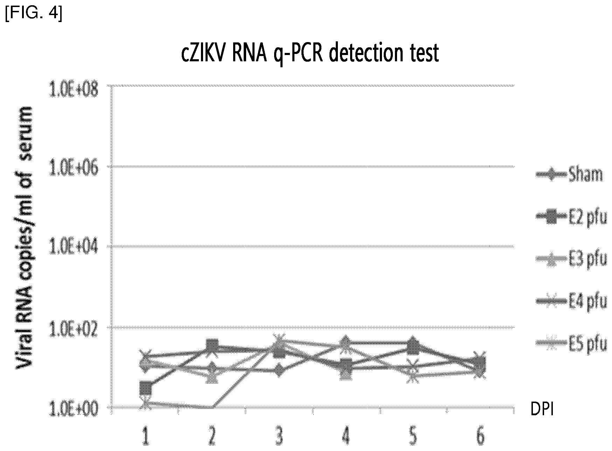

As a result, it was found that when RNA was isolated from the isolated serum and detection was performed by q-PCR, viral RNA was not detected even when cells were treated with cZIKV at various doses. Specifically, as illustrated in FIG. 4, YF17D (yellow fever 17D) and ZIKA exhibit replication ability in Balb/c, but the oncolytic virus, which is a chimeric form of the two viruses, hardly exhibits replication or proliferation ability, verifying its safety.

Meanwhile, in previous studies, ZIKV and YF 17D (yellow fever 17D) have exhibited replication ability in Balb/c. Specifically, it was reported that ZIKV wild type (PRVABC59; low-passage ZIKV isolate from Puerto Rico) proliferated to 1E6 PFU/ml on the third day after challenge and YF 17D proliferated at a high level (5E5 PFU/ml) in serum for 9 days after inoculation (Nature. 2016 Aug 25; 536(7617): 474-478., Microbiol Spectr. 2022 Oct 26;10(5): e0224622.).

Through the results above, it was verified that an oncolytic virus is a virus proven to be safe compared to other viruses (YF and ZIKA).

**Experimental Example 5. Examination of animal efficacy using three types of cell lines with different virus susceptibilities**

**5-1. Preparation of cell lines**

One vial of the cell line (MDAMB231) purchased from the Korean Cell Line Bank was thawed and passaged using RPMI1640 (FBS 10%, P/S 1%) medium. Afterwards, 3E6 cells were seeded in a 175T flask and cultured in an incubator at 37°C and 5% $CO_2$. When 100% confluency was achieved, washing with PBS was performed, 2.5% Trypsin-EDTA was added to detach the cells, centrifugation (1000 rpm, 5 min) was performed, the supernatant was discarded, and a new medium was used to obtain a cell suspension. The animal cells were prepared on the day of injection. After the viability was examined using a microscope, the cells and Matrigel were mixed at 1 : 1 and the E7 cells were finally injected into the animal.

**5-2. Transplantation of cell lines**

After the adaptation period in the breeding environment ended, the day before cell line transplantation, the backs of the mice were shaved using an electric razor. When cell lines were prepared, resuspension was performed for homogenization, and the prepared cell lines were immediately administered to animals. When the cell lines were transplanted, the left and right sides of the animal were disinfected with 70% alcohol, the cell line was administered subcutaneously at E7 cells/0.1 mL/site using a syringe equipped with a 26-gauge needle, and injection was performed on both flanks of the mouse's back.

**5-3. Design of animal experiment**

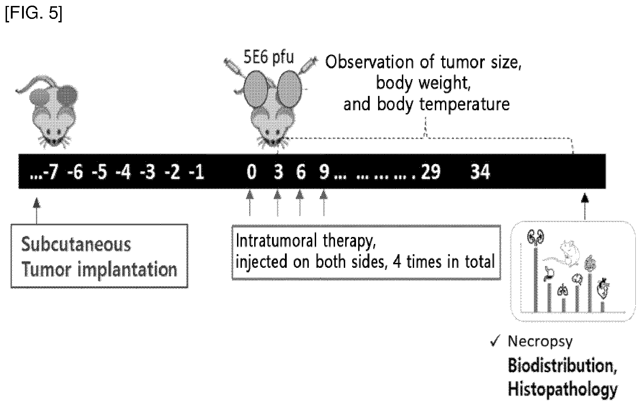

The experiment was conducted by configuring test groups as shown in Table 4 below (FIG. 5).

[Table 4]

| Group | Sex | Number of animals | Animal No. | Cell line | Substance administered | Dose (pfu/site) | Amount (μL/site) | Route of administration |
|---|---|---|---|---|---|---|---|---|
| G1 | F | 6 | 1-5 | MDA-MB-231 | Test substance | $5 \times 10^5$ | 100 | IT |
| G2 | F | 6 | 6-10 | MDA-MB-231 | PBS | - | 100 | IT |
| G3 | F | 6 | 11-15 | Hs578T | Test substance | $5 \times 10^5$ | 100 | IT |
| G4 | F | 6 | 16-20 | Hs578T | PBS | - | 100 | IT |
| G5 | F | 6 | 21-25 | BT474 | Test substance | $5 \times 10^5$ | 100 | IT |
| G6 | F | 6 | 26-30 | BT474 | PBS | - | 100 | IT |

Specifically, healthy animals were selected during the mouse acclimatization period, cell line inoculation was performed, and then the mice were ranked and divided into groups when the tumor size at the site of cell line transplantation reached about 50 to 100 mm$^3$. At this time, the mice were divided so that the tumor sizes of the respective groups were distributed as evenly as possible. The time when the tumor size reached an average of 100 mm$^3$ was designated as the starting day of administration (day 0). Afterwards, the oncolytic virus (test substance) or PBS (control group) was administered intratumorally a total of four times at three-day intervals. For intratumoral administration, animals were fixed by the dorsal skin fixation method, and intratumoral administration was performed using an insulin syringe equipped with a 31-gauge needle. During the administration and observation period, the type, date of development, and severity of general symptoms including death were observed once a day and recorded for each subject. Subjects with worsening general symptoms were isolated, and moribund and dead animals were treated as planned necropsy animals. The body weight and body temperature were measured on the day of group division, and thereafter, three times a week for four weeks. Tumor size measurement was performed three times a week for a total of 4 weeks after the starting day of test substance administration (day 0). The long and short axes of the tumor were measured using calipers, and the tumor size was calculated using the following formula.

$$\text{Tumor size} = ab2/2 \text{ (a: long axis length, b: short axis length)}$$

On the day of necropsy (day 29), all surviving animals were anesthetized, then once anesthesia was confirmed, the animals were opened and blood was collected from the posterior vena cava using a syringe. Afterwards, the abdominal aorta and posterior aorta were cut to cause bleeding/death. Thereafter, tumors were harvested from all subjects, and the spleen, brain, ovary, and liver were harvested from three subjects having the smallest tumor size compared to that of the control group in each group. The harvested tumors and organs were lightly washed with PBS, water was carefully removed therefrom using gauze, and then the tumors and organs were photographed and then weighed. The necropsy date was adjusted appropriately depending on the tumor size. One subject (total of three subjects) was selected from each control group (G2, G4, and G6), and two subjects (total of six subjects) having the smallest tumor size compared to that of the control group were selected from each treatment group (G1, G3, and G5), the tumor, ovary, spleen, brain, and liver were harvested therefrom, and then the right tumor tissue and half of the tissue were fixed in 10% neutral buffered formalin.

Homogenization (4°C, to minimize heat generation) was performed in 0.5 mL of PBS using the left tumor and 100 mg of the remaining tissue (for tissues less than 100 mg, homogenization was performed using PBS in an amount equivalent to 5 times the tissue weight). Thereafter, the homogenized tissue was centrifuged at 4°C and 13,000rpm for 10min, and the supernatant was collected, put in two tubes labeled with the animal number by 500 $\mu$L, and stored in an ultra-low temperature freezer (about -70°C). The homogenized tissues in storage were used to extract viral RNA and measure the number of viruses.

**Example 1. Test of chimeric Zika virus (cZIKV) infectivity with respect to normal cells (5 types) and various cancers (5 types)**

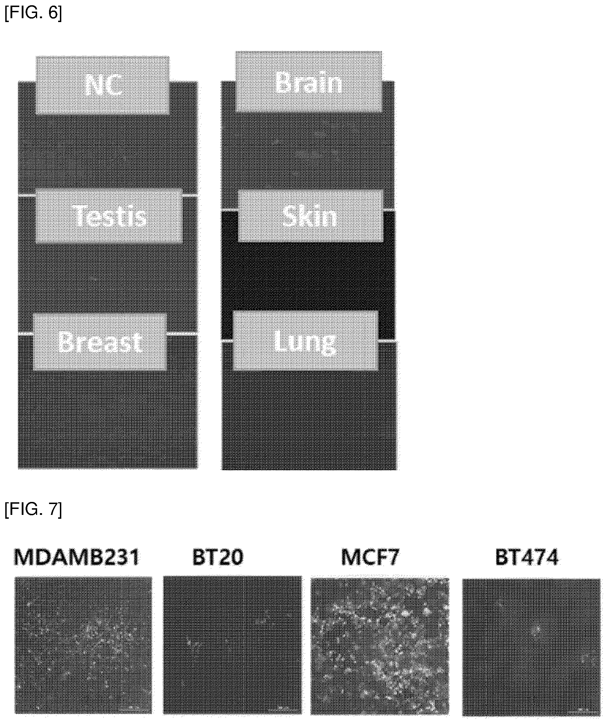

Primary cells from organs (brain, testis, skin, breast, and lung) known to be susceptible to chimeric Zika virus were purchased and the presence or absence of infectivity was examined. In order to confirm the infectivity assessment, an immunofluorescence assay was performed after virus infection. As a result, the chimeric Zika virus did not exhibit infectivity in five types of normal cells (FIG. 6).

**Example 2. Examination of infectivity depending on breast cancer subtype**

Breast cancer subtypes are classified into four types based on the presence or absence of estrogen receptors, progesterone receptors, and HER2 receptors. The four subtypes are Luminal A, Luminal B, HER2, and Triple negative. The Triple negative (basal-like) subtype, which lacks the three receptors, is known to have the worst prognosis.

Cell lines corresponding to Luminal A, Luminal B, HER2, and Triple negative according to the four subtypes were prepared, and the oncolytic virus infectivity with respect to these cell lines was examined. The immunofluorescence assay (IFA) was performed, and as a result, it was found that the oncolytic virus infected all of the Triple negative subtypes (MDAMB231 and BT20), Luminal A subtype (MCF7), and Luminal B subtype (BT474) (FIG. 7).

**Example 3. Adaptation to increase oncolytic virus infectivity and characterization of oncolytic virus**

In order to obtain a broadly effective virus that can infect various cancer cells, the oncolytic virus was repeatedly passaged in cancer cells. First, the MDAMB231 cell line in which the oncolytic virus exhibited strong infectivity and CPE was passaged for 19 passages (MDA19) or more. Afterwards, the MDA19 virus was further passaged in a cell mixture. This cell mixture consisted of cell lines in which the oncolytic virus exhibited low or no infectivity and CPE, and passaging was performed for 61 passages (Bx61) or more.

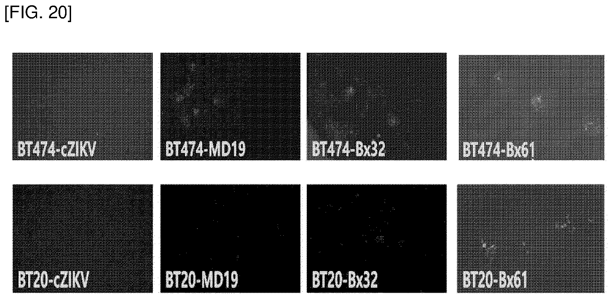

When the chimeric Zika virus was passaged in breast cancer cells, it was found that the initial chimeric Zika virus (cZIKV) had a weak level of infectivity with respect to cancer cells, but as the number of passages increased, the infectivity with respect to breast cancer cells (for example, BT20 and BT474) increased. Specifically, as illustrated in FIG. 20, when the infectivity was examined by passage through an assay, it was found that the virus infectivity with respect to specific cells (BT20 and BT474) increased as the number of passages increased (FIG. 20).

### 3-1. Comparison of infectivity

In order to characterize the oncolytic virus (adapted virus, Bx61) that had undergone passaging, an experiment to compare the infectivity and CPE of the oncolytic virus with those of the parental virus (original virus, cZIKV) was conducted. For comparison of infectivity, the expression level of the receptor AXL, which Zika virus uses for infection, was also investigated, and the correlation was illustrated in the graph.

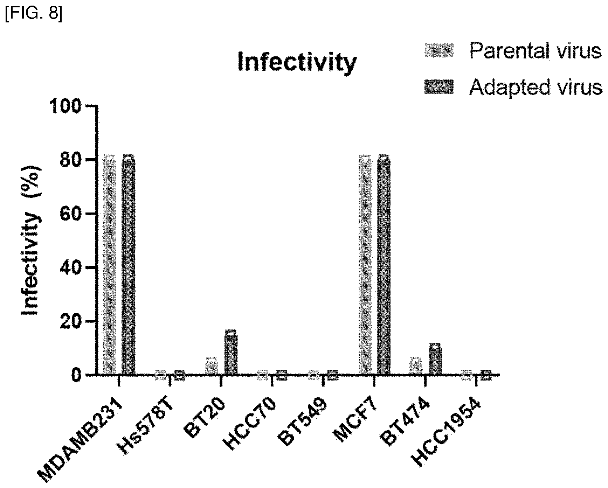

As a result, as illustrated in FIG. 8, the infectivity was high in two types of cell lines, MDAMB231 and MCF7, the infectivity was low in BT20 and BT474, and the infectivity was hardly detected in the remaining cell lines. The expression rate of AXL did not show any correlation with the infectivity.

### 3-2. Examination of cytotoxicity using ToxGlo assay

In order to examine whether the oncolytic virus infected cell lines and exhibited cytotoxicity, the ToxGlo assay was performed on six types of cell lines as illustrated in the figure.

Vero cells that were known as an existing production cell line, two types of cell lines (MCF7 and MDAMB231) that were easily infected, a cell line (BT20) that was hardly infected, a cell line (Hs578T) that was not infected, and normal cells (HMEc) were tested.

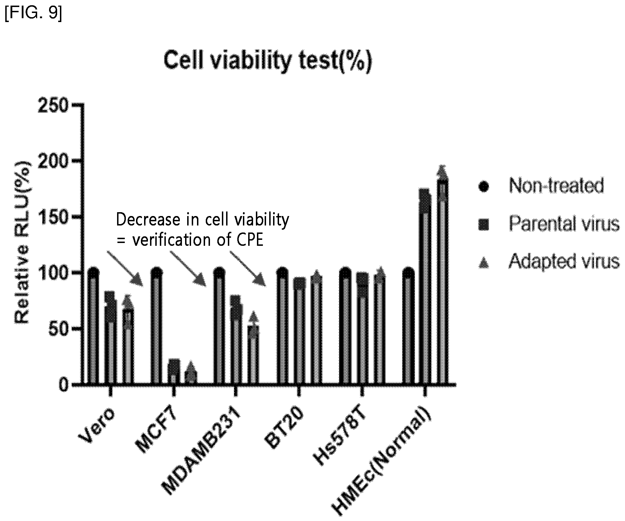

As a result, as illustrated in FIG. 9, CPE was strongly exhibited in the Vero cells and MCF7 and MDAMB231 cells when the cells were infected with the virus. In these cells, it was found that the CPE was higher than that of the parental virus by about 10% when the cells were infected with the adapted virus. On the other hand, in the cell lines that were hardly or not infected with the virus and in normal cells, there was no significant change in cell line viability due to virus infection. In normal cells, cell proliferation actually increased.

### 3-3. Full-length sequence analysis of MDA19 and Bx61 viruses undergone passaging

In order to examine whether there was any variation in the MDA19 and Bx61 viruses that had undergone passaging, full-length sequence analysis was performed. The results are as shown in Table 5 below. In the case of MDA19, a total of 27 amino acid variations occurred, and among these, the total number of amino acid variations in the prM-E protein, which was the site involved in infection, was eight. In the case of Bx61, a total of 20 amino acid variations occurred, and among these, the total number of amino acid variations in the prM-E protein was seven.

[Table 5]

| | Capsid | prM | M | E | Non-structural |
|---|---|---|---|---|---|
| MDA19 | R75T | A122V | R245K | K455E | L1598M,A1600T,A1820T, I1843M,E2038D,N2042K, D2044Y,K2055N,S2079T, K2054N,R2122K,V2129I, G2142S,R2409K,E2416K, A2456v,H2503Y |
| | R98T | E142D | | S657I | |
| | | S171N | | H690Y | |
| | | E208G | | | |
| | 2 | 4 | 1 | 3 | 17 |
| Bx61 | K30E, K60R | A122V | Q227R | R427Q N497K A608T S657T H690Y | D1464N,I1843M,Q2052H, Q2108H,A2139V,S2150N, F2350L,K2404R, E2416K, P2461S,N3077I |
| | 2 | 1 | 1 | 5 | 11 |

**Example 4. Animal efficacy experiment using three types of cells with different virus susceptibilities**

The following animal efficacy experiment was conducted using the oncolytic virus Bx61.

**4-1. Xenograft experiment using MDAMB231, a cell line with high susceptibility to oncolytic virus**

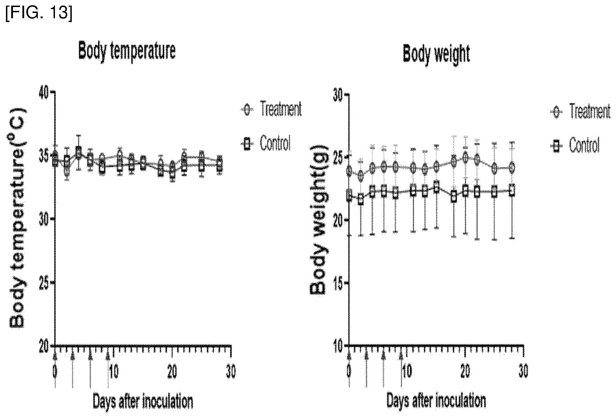

In order to evaluate the anticancer effect of the oncolytic virus, the MDAMB231 cell line was engrafted into Balb/c nude mice lacking T cells. After a xenograft model was constructed by subcutaneously transplanting cells to the left and right sides, the oncolytic virus was administered intratumorally, and then the mice were observed for 28 days. As a result of observing general symptoms, there was no dead subject during the experimental period, and abnormal symptoms due to the administration of the oncolytic virus were not observed. There was no significant change in body weight or body temperature in either the oncolytic virus administration group or the control group during the test period (FIG. 13).

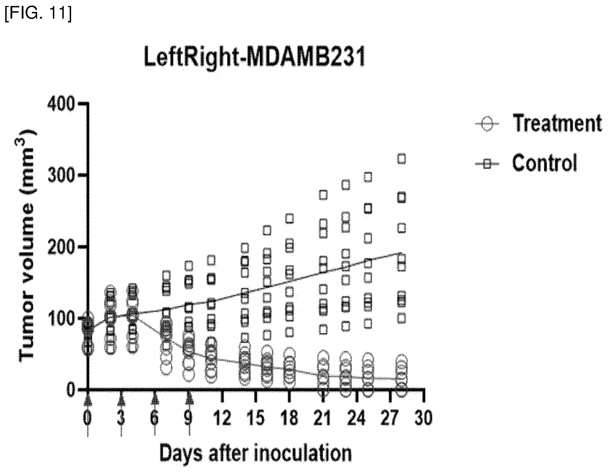

As a result of measuring the tumor size, the tumor size of the treatment group (G1) was significantly smaller than that of the control group (G2) from day 7 to day 28 after the start of oncolytic virus administration. It was found that the tumor size began to decrease from the second administration of the oncolytic virus and the tumor gradually disappeared (FIG. 11).

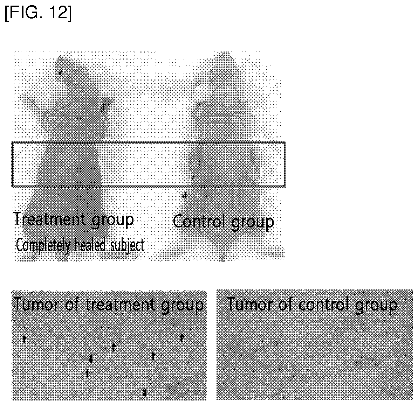

As a result of histopathological examination of tumors, central necrosis was observed in the tumors of the oncolytic virus treatment group and inflammatory cell infiltration and fibrosis were additionally observed. On the other hand, in the case of the tumor tissue of the control group, tumor cells were maintained at the edge and formed a tumor (FIG. 12).

**4-2. Xenograft experiment using BT474, a cell line with low susceptibility to oncolytic virus**

In order to evaluate the anticancer effect of the oncolytic virus, the BT474 cell line was engrafted into Balb/c nude mice lacking T cells. After a xenograft model was constructed by subcutaneously transplanting cells to the left and right sides, the oncolytic virus was administered intratumorally, and then the mice were observed for 28 days. As a result of observing general symptoms, there was no dead subject during the experimental period, and abnormal symptoms due to the administration of the oncolytic virus were not observed. There was no significant change in body weight or body temperature in either the oncolytic virus administration group or the control group during the test period, and the body weight of the treatment group was actually higher by about 2 g on average (FIG. 13).

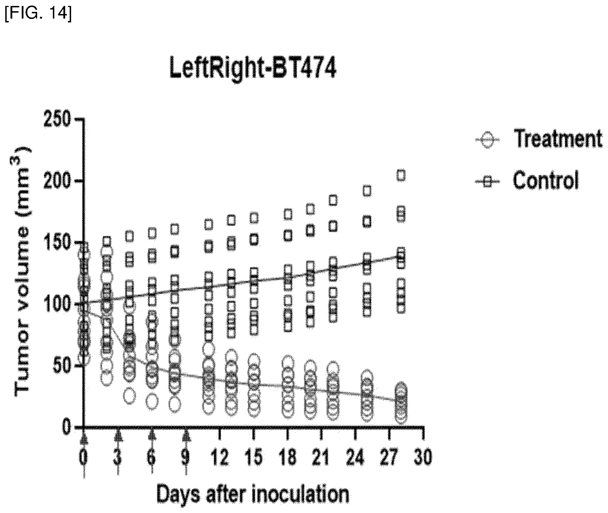

As a result of measuring the tumor size, the tumor size of the treatment group (G3) was significantly smaller than that of the control group (G4) from day 4 to day 28 after the start of oncolytic virus administration. It was found that the tumor size began to decrease from the second administration of the oncolytic virus, and the difference in tumor size between the groups gradually increased as the tumor size decreased compared to that of the control group (FIG. 14).

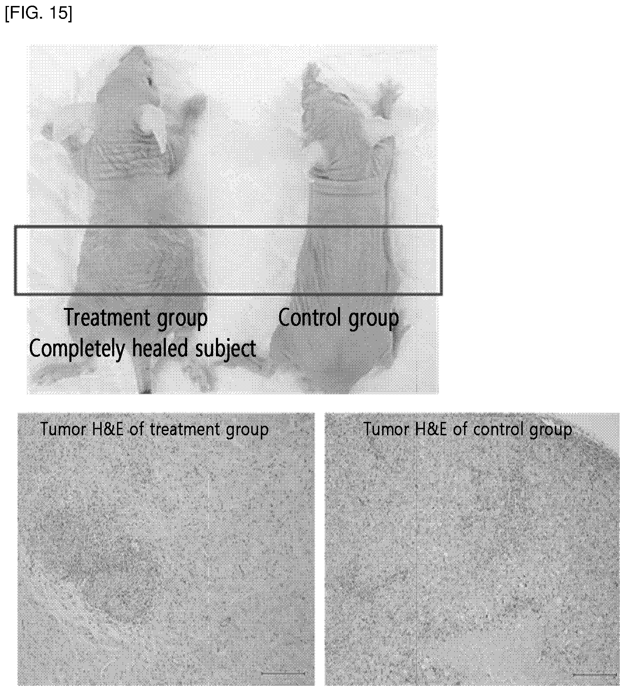

As a result of histopathological examination of tumors, central necrosis was observed in the tumors of the oncolytic virus treatment group and inflammatory cell infiltration and fibrosis were additionally observed. On the other hand, in the case of the tumor tissue of the control group, tumor cells were maintained at the edge and formed a tumor (FIG. 15).

**4-3. Xenograft experiment using Hs578T, a cell line with no CPE to oncolytic virus**

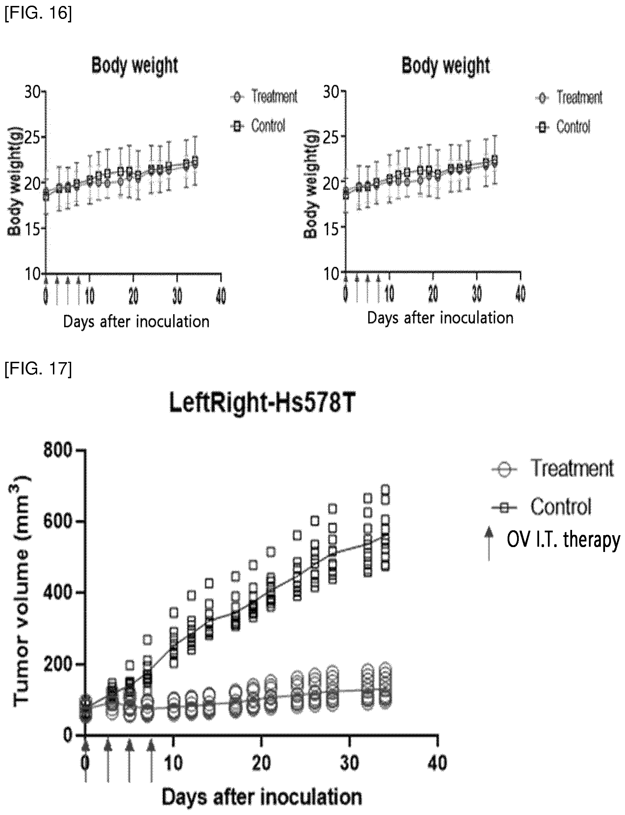

In order to evaluate the anticancer effect of the oncolytic virus, the Hs578T cell line was engrafted into Balb/c nude mice lacking T cells. After a xenograft model was constructed by subcutaneously transplanting cells to the left and right sides, the oncolytic virus was administered intratumorally, and then the mice were observed for 28 days. As a result of observing general symptoms, , there was no dead subject during the experimental period, and abnormal symptoms due to the administration of the oncolytic virus were not observed. There was no significant change in body weight or body temperature in either the oncolytic virus administration group or the control group during the test period (FIG. 16).

As a result of measuring the tumor size, the tumor size of the treatment group (G5) was significantly smaller than that of the control group (G6) from day 6 to day 32 after the start of oncolytic virus administration. It was found that the growth rate of tumor size significantly decreased compared to that of the control group from the second administration of the oncolytic virus and the difference in tumor size between the groups increased (FIG. 17).

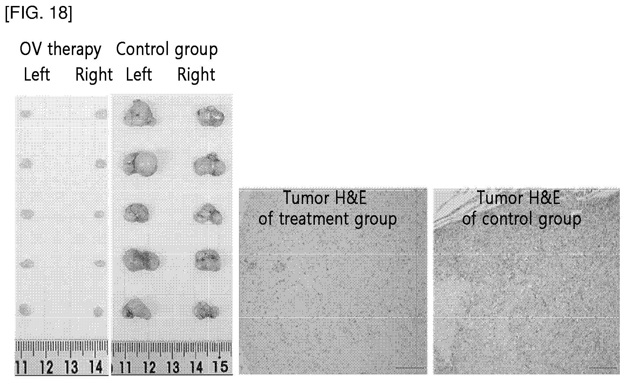

As a result of histopathological examination of tumors, a small number of tumor cells were observed in the tumor of the oncolytic virus treatment group, and fibrosis was also observed. This suggests that part of the tumor disappeared or was replaced with fibrous tissue after administration of the oncolytic virus, indicating that the oncolytic virus affected the inhibition of tumor cell proliferation (FIG. 18).

**Example 5. Safety verification of treatment using oncolytic virus through histopathology result**

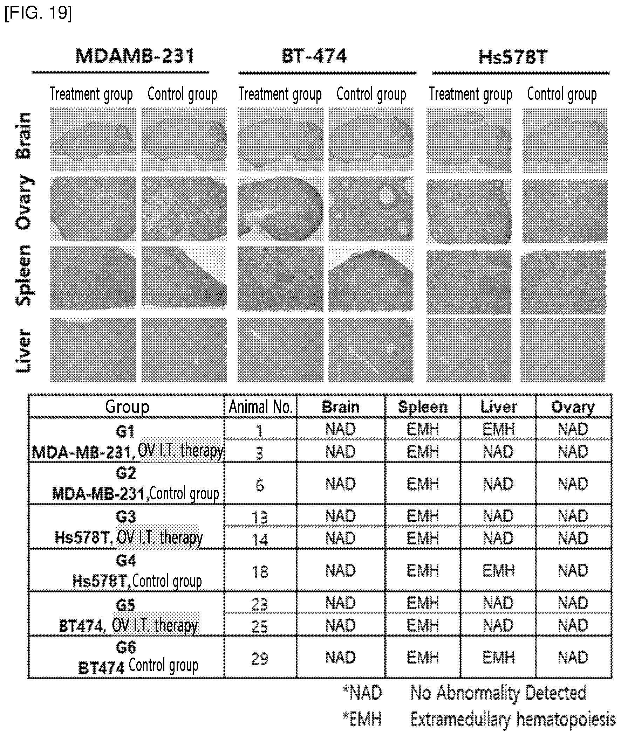

Histopathological examination of major organs was conducted. H&E staining was performed to select major organs that were susceptible to the oncolytic virus and determine whether there were lesions in the selected major organs. As a result, abnormalities were not observed in the brain and ovaries of all the groups (FIG. 19). Extramedullary hematopoiesis was observed in all spleen slides and some liver slides, but these lesions are background lesions commonly observed in mice. Therefore, the safety of the oncolytic virus was proven since inflammatory response and abnormal pathology were not observed in major organs.

From the above description, those skilled in the art to which the present invention pertains will understand that the present invention can be implemented in other specific forms without changing the technical idea or essential characteristics thereof. In this regard, it should be understood that the embodiments described above are illustrative in all respects but not restrictive. The scope of the present invention is defined by the appended claims rather than by the description preceding them, and all changes or modifications derived from the meaning and scope of the claims and their equivalent concepts should be construed as being included in the scope of the present invention.

**Claims**

1. A pharmaceutical composition for prevention or treatment of breast cancer, comprising a chimeric Zika virus formed by introducing a Zika virus antigen gene into a gene of an attenuated yellow fever virus containing SEQ ID NO: 1 as an active ingredient.

2. The composition according to claim 1, wherein the Zika virus antigen gene includes i) a gene encoding a pre-membrane (prM) protein and ii) a gene encoding an envelope (E) protein.

3. The composition according to claim 1, wherein the Zika virus antigen gene includes i) a gene encoding a pre-membrane (prM) protein of SEQ ID NO: 3 or having 90% or more identity to SEQ ID NO: 3; and ii) a gene encoding an envelope (E) protein of SEQ ID NO: 4 or having 90% or more identity to SEQ ID NO: 4.

4. The composition according to claim 1, wherein the chimeric Zika virus has ability of specifically inhibiting breast cancer cells through continuous passaging.

5. The composition according to claim 4, wherein the chimeric Zika virus is obtained through at least 19 passages in MDAMB-231 cells and at least 61 continuous passages in mixed cells consisting of HCC1954, BT474, HCC70, BT20, BT549, and HS578T.

6. The composition according to claim 5, wherein in the chimeric Zika virus, an amino acid corresponding to any one position in the group consisting of 30th, 60th, 122nd, 227th, 427th, 497th, 608th, 657th, 690th, 1464th, 1843rd, 2052nd, 2108th, 2139th, 2150th, 2350th, 2404th, 2416th, 2461st, and 3077th positions based on a sequence represented by SEQ ID NO: 7 is substituted with another amino acid.

7. The composition according to claim 5, wherein the chimeric Zika virus includes a polypeptide described by an amino acid sequence of SEQ ID NO: 8 or having 90% or more identity to SEQ ID NO: 8.

8. The composition according to claim 5, wherein the chimeric Zika virus includes a gene encoding a capsid protein of yellow fever virus and a gene encoding a non-structural protein.

9. The composition according to claim 8, wherein the capsid (C) protein includes a polypeptide described by an amino acid sequence of SEQ ID NO: 2 or having 90% or more identity to SEQ ID NO: 2.

10. The composition according to claim 9, wherein in the chimeric Zika virus, amino acids corresponding to 30th and 60th positions based on a sequence represented by SEQ ID NO: 2 are substituted with other amino acids.

11. The composition according to claim 5, wherein a pre-membrane (prM) protein of the chimeric Zika virus includes a polypeptide described by an amino acid sequence of SEQ ID NO: 3 or having 90% or more identity to SEQ ID NO: 3.

12. The composition according to claim 11, wherein in the chimeric Zika virus, amino acids corresponding to 122th and 227th positions based on a sequence represented by SEQ ID NO: 3 are substituted with other amino acids.

13. The composition according to claim 5, wherein an envelope (E) protein of the chimeric Zika virus includes a polypeptide described by an amino acid sequence of SEQ ID NO: 4 or having 90% or more identity to SEQ ID NO: 4.

14. The composition according to claim 13, wherein in the chimeric Zika virus, an amino acid corresponding to any one position in the group consisting of 427th, 497th, 608th, 657th, and 690th positions based on a sequence represented by SEQ ID NO: 4 is substituted with another amino acid.

15. The composition according to claim 8, wherein the non-structural protein includes a polypeptide described by an amino acid sequence of SEQ ID NO: 5 or having 90% or more identity to SEQ ID NO: 5.

16. The composition according to claim 15, wherein in the chimeric Zika virus, amino acids corresponding to 1464th, 1843rd, 2052nd, 2108th, 2139th, 2150th, 2350th, 2404th, 2416th, 2461st, and 3077th positions based on a sequence represented by SEQ ID NO: 5 are substituted with other amino acids.

17. The composition according to claim 1, wherein the composition is administered intratumorally, intramuscularly, intraperitoneally, or intravenously.

18. A method for preparing a composition for prevention or treatment breast cancer, the method comprising:

(a) continuously subculturing a chimeric Zika virus in MDAMB-231 cells at least 19 times to obtain a virus culture containing a cell-adapted virus;
(b) continuously subculturing the culture containing a cell-adapted virus, which is obtained in step (a), in mixed cells consisting of HCC1954, BT474, HCC70, BT20, BT549, and HS578T at least 61 times to obtain a virus culture containing a cell-adapted virus; and
(c) selecting and isolating a chimeric Zika virus from the virus culture containing a cell-adapted virus, which is obtained in step (b), by evaluating ability of specifically inhibiting breast cancer cells.

19. A chimeric nucleic acid molecule consisting of i) a gene encoding a signal peptide located upstream of a gene encoding a pre-membrane (prM) protein including a polypeptide of SEQ ID NO: 6 or having 90% or more identity to SEQ ID NO: 6; ii) a gene encoding a pre-membrane (prM) protein including a polypeptide of SEQ ID NO: 3 or having 90% or more identity to SEQ ID NO: 3; or iii) a gene encoding an envelope (E) protein consisting of a polypeptide of SEQ ID NO: 4 or having 90% or more identity to SEQ ID NO: 4 of Zika virus, which is inserted in place of

a yellow fever virus 17D vaccine strain gene into regions of i) a gene encoding a signal peptide located upstream of a gene encoding a pre-membrane (prM) protein; ii) a gene encoding a pre-membrane (prM) protein; and iii) a gene encoding an envelope (E) protein of a yellow fever virus 17D vaccine strain nucleic acid molecule, wherein part or all of a nucleic acid constituting the gene i), ii), or iii) of Zika virus is substituted, deleted, or inserted.

20. A chimeric Zika virus mutant strain obtained by continuously subculturing the chimeric nucleic acid molecule according to claim 19 in breast cancer cells to have increased cancer cell adaptability.

[FIG. 1]

Zika virus prM-E protein Substitution

Main antigen protein

cZIKV 5` UTR | C | prM | E-TM | E | E-TM | NS1 | NS2 | NS3 | NS4 | NS5 | 3` UTR

Structural protein

Non-structural protein

Yellow fever genome

[FIG. 2]

[FIG. 3]

ATP Detection Substrate

ATP Detection Buffer

ATP Detection Reagent

Mix

Luminometer

11320MA

[FIG. 4]

cZIKV RNA q-PCR detection test

[FIG. 5]

5E6 pfu

Observation of tumor size,
body weight,
and body temperature

...-7 -6 -5 -4 -3 -2 -1    0   3  6  9... ... ... ... . 29    34

Subcutaneous
Tumor implantation

Intratumoral therapy,
injected on both sides, 4 times in total

✓ Necropsy
**Biodistribution,
Histopathology**

[FIG. 6]

[FIG. 7]

MDAMB231　　BT20　　MCF7　　BT474

[FIG. 8]

**Infectivity**

Parental virus
Adapted virus

[FIG. 9]

Cell viability test(%)

[FIG. 10]

Body weight

Body temperature

[FIG. 11]

LeftRight-MDAMB231

[FIG. 12]

[FIG. 13]

Body temperature

Body weight

[FIG. 14]

LeftRight-BT474

[FIG. 15]

Treatment group    Control group
Completely healed subject

Tumor H&E of treatment group

Tumor H&E of control group

[FIG. 16]

[FIG. 17]

[FIG. 18]

[FIG. 19]

| Group | Animal No. | Brain | Spleen | Liver | Ovary |
|---|---|---|---|---|---|
| G1 MDA-MB-231, OV I.T. therapy | 1 | NAD | EMH | EMH | NAD |
| | 3 | NAD | EMH | NAD | NAD |
| G2 MDA-MB-231, Control group | 6 | NAD | EMH | NAD | NAD |
| G3 Hs578T, OV I.T. therapy | 13 | NAD | EMH | NAD | NAD |
| | 14 | NAD | EMH | NAD | NAD |
| G4 Hs578T, Control group | 18 | NAD | EMH | EMH | NAD |
| G5 BT474, OV I.T. therapy | 23 | NAD | EMH | NAD | NAD |
| | 25 | NAD | EMH | NAD | NAD |
| G6 BT474 Control group | 29 | NAD | EMH | EMH | NAD |

*NAD   No Abnormality Detected
*EMH   Extramedullary hematopoiesis

[FIG. 20]

<div align="center">**INTERNATIONAL SEARCH REPORT**</div>

| International application No. |
| --- |
| **PCT/KR2023/005736** |

| A. | CLASSIFICATION OF SUBJECT MATTER |
| --- | --- |

**A61K 35/768**(2015.01)i; **A61K 39/12**(2006.01)i; **A61P 35/00**(2006.01)i; **C12N 7/00**(2006.01)i; **C07K 14/005**(2006.01)i; **A61K 39/00**(2006.01)i

According to International Patent Classification (IPC) or to both national classification and IPC

| B. | FIELDS SEARCHED |
| --- | --- |

Minimum documentation searched (classification system followed by classification symbols)

A61K 35/768(2015.01); A61K 39/00(2006.01); A61K 39/12(2006.01); A61K 39/39(2006.01); C07K 14/005(2006.01); C07K 16/28(2006.01); C12N 7/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 키메릭 지카바이러스(chimeric ZIKA virus), 황열 바이러스(yellow fever virus), 유방암(breast cancer)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| DY | KR 10-2019-0127605 A (SK BIOSCIENCE CO., LTD.) 13 November 2019 (2019-11-13)<br>See abstract; paragraphs [0061], [0192] and [0279]; figures 2-3; SEQ ID NOs: 1 and 13; and claims 1, 5-6 and 11-18. | 1-17,19-20 |
| DA | | 18 |
| Y | US 2019-0038685 A1 (THE NEMOURS FOUNDATION) 07 February 2019 (2019-02-07)<br>See paragraph [0019]; and claims 1-2 and 9. | 1-18 |
| Y | US 10967057 B2 (GLAXOSMITHKLINE BIOLOGICALS, S.A.) 06 April 2021 (2021-04-06)<br>See column 3, lines 44-46; and SEQ ID NO: 2. | 3,11-14,19-20 |
| Y | RODRÍGUEZ STEWART, R. M. et al. Enhanced killing of triple-negative breast cancer cells by reassortant reovirus and topoisomerase inhibitors. Journal of virology. 2019, vol. 93, no. 23, thesis no.: e01411-19, pp. 1-21.<br>See abstract; figure 1; and pages 2-4 and 13. | 4-5,18,20 |

☑ Further documents are listed in the continuation of Box C.   ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **07 August 2023** | **07 August 2023** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

International application No.

**PCT/KR2023/005736**

### C.    DOCUMENTS CONSIDERED TO BE RELEVANT

| Category* | Citation of document, with indication, where appropriate, of the relevant passages | Relevant to claim No. |
|---|---|---|
| Y | WO 2018-060771 A1 (SANOFI PASTEUR) 05 April 2018 (2018-04-05)<br>See abstract; and claims 1-24. | 1-17 |
| A | KR 10-2253190 B1 (GENEMATRIX INC.) 18 May 2021 (2021-05-18)<br>See entire document. | 1-20 |

Form PCT/ISA/210 (second sheet) (July 2022)

**INTERNATIONAL SEARCH REPORT**

International application No.

**PCT/KR2023/005736**

| Box No. I | Nucleotide and/or amino acid sequence(s) (Continuation of item 1.c of the first sheet) |

1.  With regard to any nucleotide and/or amino acid sequence disclosed in the international application, the international search was carried out on the basis of a sequence listing:

    a.  ☑  forming part of the international application as filed.

    b.  ☐  furnished subsequent to the international filing date for the purposes of international search (Rule 13*ter*.1(a)),

        ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

**INTERNATIONAL SEARCH REPORT**
Information on patent family members

International application No.

**PCT/KR2023/005736**

| Patent document cited in search report | | | Publication date (day/month/year) | Patent family member(s) | | | Publication date (day/month/year) |
|---|---|---|---|---|---|---|---|
| KR | 10-2019-0127605 | A | 13 November 2019 | WO | 2019-212312 | A1 | 07 November 2019 |
| US | 2019-0038685 | A1 | 07 February 2019 | US | 10993975 | B2 | 04 May 2021 |
| | | | | US | 2021-0322495 | A1 | 21 October 2021 |
| US | 10967057 | B2 | 06 April 2021 | BE | 1024796 | A1 | 02 July 2018 |
| | | | | BE | 1024796 | A9 | 03 January 2019 |
| | | | | BE | 1024796 | B1 | 10 July 2018 |
| | | | | EP | 3463445 | A1 | 10 April 2019 |
| | | | | US | 2019-0134184 | A1 | 09 May 2019 |
| | | | | WO | 2017-208191 | A1 | 07 December 2017 |
| WO | 2018-060771 | A1 | 05 April 2018 | | None | | |
| KR | 10-2253190 | B1 | 18 May 2021 | KR | 10-2020-0010036 | A | 30 January 2020 |
| | | | | WO | 2020-017765 | A1 | 23 January 2020 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020190127605

**Non-patent literature cited in the description**

- **JAGUS R et al.** *Int. J. Biochem. Cell Biol.*, 1999, vol. 31, 123-138
- **BISCHOFF JR et al.** *Science*, 1996, vol. 274 (5286), 373-376
- **PEARSON et al.** *Proc. Natl. Acad. Sci. USA*, 1988, vol. 85, 2444
- **NEEDLEMAN** ; **WUNSCH**. *J. Mol. Biol.*, 1970, vol. 48, 443-453
- **RICE et al.** EMBOSS: The European Molecular Biology Open Software Suite. *Trends Genet.*, 2000, vol. 16, 276-277
- **DEVEREUX, J. et al.** *Nucleic Acids Research*, 1984, vol. 12, 387
- **ATSCHUL, [S.] [F.** *J MOLEC BIOL*, 1990, vol. 215, 403
- Guide to Huge Computers. Academic Press, 1994
- **CARILLO**. *SIAM J Applied Math*, 1988, vol. 48, 1073
- **NEEDLEMAN et al.** *J Mol Biol.*, 1970, vol. 48, 443
- **SMITH** ; **WATERMAN**. *Adv. Appl. Math*, 1981, vol. 2, 482
- **GRIBSKOV et al.** *Nucl. Acids Res.*, 1986, vol. 14, 6745
- Atlas Of Protein Sequence And Structure. National Biomedical Research Foundation, 1979, 353-358
- *Nature*, 25 August 2016, vol. 536 (7617), 474-478
- *Microbiol Spectr.*, 26 October 2022, vol. 10 (5), e0224622